Europäisches Patentamt

European Patent Office

Office européen des brevets

(11) Numéro de publication : **0 520 916 A1**

# (12) DEMANDE DE BREVET EUROPEEN

(21) Numéro de dépôt : **92420220.3**

(22) Date de dépôt : **26.06.92**

(51) Int. Cl.⁵ : **A45C 5/12**

(30) Priorité : **27.06.91 FR 9108194**

(43) Date de publication de la demande :
**30.12.92 Bulletin 92/53**

(84) Etats contractants désignés :
**AT BE CH DE DK ES FR GB GR IT LI LU MC NL PT SE**

(71) Demandeur : **Simon, Nicholas**
**1 Bd Maréchal Joffre**
**F-38000 Grenoble (FR)**

(72) Inventeur : **Simon, Nicholas**
**1 Bd Maréchal Joffre**
**F-38000 Grenoble (FR)**

(74) Mandataire : **Guerre, Dominique et al**
**Cabinet Germain et Maureau 20 Boulevard Eugène Deruelle BP 3011**
**F-69392 Lyon Cédex 03 (FR)**

(54) **Nécessaire de transport.**

(57)    Necéssaire de transport de différents produits contenus respectivement dans différents emballages (1) unitaires, par exemple en carton, chaque emballage unitaire comprenant une face de présentation (1a) du produit contenu, par exemple une face informative, ledit nécessaire comprenant un contenant (2) dont l'intérieur est accessible au chargement avec les différents produits emballés, grâce à un moyen (3) d'ouverture-fermeture dudit contenant, la surface interne dudit contenant comprenant de manière non détachable des moyens (4) de fixation réversible, tels qu'auto-agrippants, caractérisé en ce que le nécessaire comporte avant utilisation une pluralité d'éléments complémentaires (5) de fixation réversible, tels que pastilles, distincts du contenant (2), comportant chacun d'un côté une face (5a) d'adhésion à la surface d'un emballage (1) unitaire, protégée par un film de protection (6) non adhésif, et de l'autre côté, une face (5b) de fixation réversible, telle qu'auto-agrippante, sur les moyens (2) de fixation réversible du contenant.

FIG 4

La présente invention concerne de manière générale les contenants, tels que valises, mallettes, ou trousses, permettant de transporter différents objets à usage personnel ou professionnel, avec une fixation réversible desdits objets dans ledit contenant, permettant notamment leur rangement dans ce dernier.

Conformément au document FR-A-2 653 312, on a décrit une valise ou similaire, à usage personnel, dont l'intérieur est accessible au chargement avec différents objets, en l'occurrence des pochettes ou trousses, pouvant elles-mêmes contenir d'autres objets, tels que boîtes ou instruments. Cette accessibilité résulte de l'existence sur la valise d'au moins un moyen d'ouverture-fermeture, tel qu'une enveloppe en deux parties, reliées entre elles par une articulation, et avec une fermeture à glissière le long des deux bordures adjacentes desdites parties. Tout ou partie de la surface interne de la valise comprend, de manière non détachable, différents moyens de fixation réversible, tels qu'auto-agrippants, par exemple un tapis textile dont la face extérieure présente une multiplicité d'éléments non visibles à l'oeil nu, du type boucles. Complémentairement aux moyens de fixation réversible de la valise, la paroi postérieure des pochettes ou trousses présente des moyens de fixation réversible, destinés à coopérer avec ceux à l'intérieur de la valise, tels qu'auto-agrippants, par exemple une bande textile dont la face extérieure présente une multiplicité d'éléments non visibles à l'oeil nu, du type crochets.

Une telle valise apparaît inadaptée à un usage professionnel, en présence des exigences suivantes :

1°) toujours de manière fixée réversiblement dans le contenant, à l'intérieur et sur ses parois, permettre le transport des objets et produits les plus divers, allant de simples boîtes aux instruments les plus variés

2°) éviter si possible le déconditionnement des objets transportés, lorsque ceux-ci consistent en des produits contenus dans un emballage unitaire, de manière à bénéficier en permanence des différentes informations présentes sur ledit emballage

3°) permettre un rangement effectif des objets transportés, sans cloisonnement rapporté à l'intérieur du contenant

4°) permettre une présentation des objets transportés à la vue de l'utilisateur, dès l'ouverture du contenant, assurant une visibilité et une distinctivité optimales desdits objets, notamment avec une bonne lisibilité des informations écrites, présentes à l'extérieur desdits objets.

Ces exigences sont en particulier celles requises pour une trousse médicale d'urgence.

La présente invention a pour objet une solution permettant de satisfaire à l'ensemble des ces exigences.

Selon la présente invention, on propose un nécessaire, ensemble ou kit, associant avant utilisation, outre un contenant tel que précédemment décrit, une pluralité d'éléments complémentaires de fixation réversible, tels que pastilles, distincts du contenant. Chaque dit élément complémentaire comporte, d'un côté une face d'adhésion, c'est-à-dire susceptible de coller ou d'être collée, protégée temporairement par un film de protection non adhésif, et de l'autre côté une face de fixation réversible, telle qu'auto-agrippante, sur les moyens de fixation réversible du contenant.

Chaque élément ou pastille complémentaire de fixation réversible, est plus particulièrement destiné, mais pas exclusivement, à être fixé sur une face d'un emballage unitaire (par exemple en carton) contenant un produit, opposée à la face informative ou de présentation dudit emballage. Il suffit pour cela de détacher ou peler le film de protection de la pastille, et de la coller par sa face adhésive sur ladite face opposée de l'emballage unitaire. On obtient ainsi un emballage unitaire contenant le produit, par exemple un flacon de médicament, pouvant être fixé directement, de manière réversible, dans le contenant. Et chaque emballage unitaire est alors fixé directement, de manière réversible, par la face de fixation réversible de la pastille, par exemple auto-agrippante, sur les moyens de fixation réversible du contenant.

Bien entendu, les mêmes éléments complémentaires de fixation réversible, tels que pastilles, peuvent être mis en oeuvre, exactement à la manière décrite précédemment, pour fixer de manière réversible dans le contenant, autre chose qu'un emballage unitaire, par exemple un instrument.

Appliquée à l'art médical ou chirurgical, la présente invention assure une lisibilité quasi-parfaite et sûre, et du contenu de la valise ou mallette, et des informations relatives aux objets contenus, telles que prescriptions d'utilisation, dates de péremption, mentions légales, posologie, etc... Cette lisibilité immédiate et sûre est particulièrement importante pour des interventions médicales urgentes, de secours.

La présente invention permet de ranger des boîtes côte-à-côte, ayant les dimensions les plus diverses, pour obtenir un présentoir informatif en quelque sorte, rassemblant sur une même face toutes les informations utiles des différents produits rangés. L'invention incite aussi au rangement systématique, d'une boîte donnée, toujours au même endroit, correspondant au trou laissé libre par l'extraction de ladite boîte du présentoir informatif.

La présente invention est maintenant décrite par référence aux dessins annexés, dans lesquels :

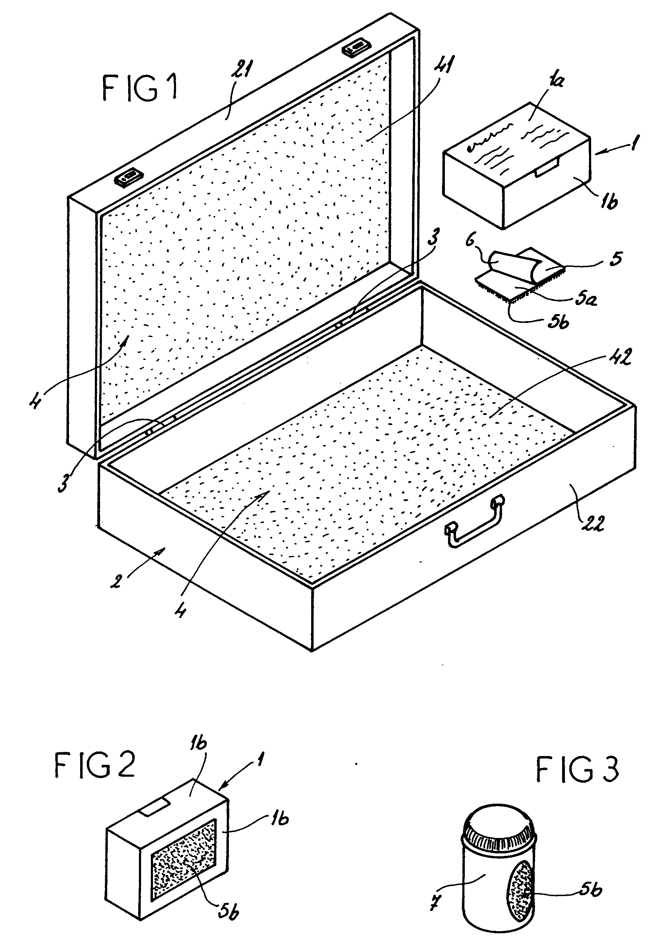

∗ la Figure 1 représente un nécessaire de transport selon l'invention, avant son utilisation

∗ la Figure 2 représente un emballage unitaire, prêt à être fixé de manière réversible dans le contenant, par exemple une valise, représenté à la Figure 1

* la Figure 3 représente un autre type d'emballage unitaire, prêt à être fixé de manière réversible dans la valise de la Figure 1

* la Figure 4 représente un nécessaire de transport selon l'invention, tel qu'utilisé.

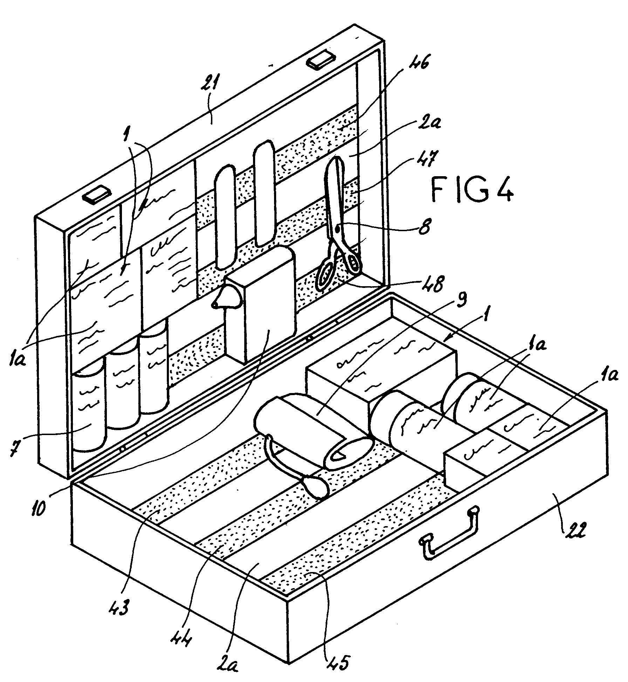

Le nécessaire de transport représenté aux Figures 1 et 4 comprend avant son utilisation :

- un contenant 2, du type valise ou mallette, en deux parties 21 et 22 articulées l'une sur l'autre par des charnières 3, ce qui permet d'ouvrir et fermer la valise, et d'accéder à l'intérieur de cette dernière

- la surface interne de cette valise comprend à demeure, c'est-à-dire de manière non détachable, deux tapis 41 et 42 de fixation réversible, auto-agrippants, recouvrant la face interne des deux parties 21 et 22 de la valise respectivement; par exemple chacun de ces tapis de nature textile présente une surface extérieure constituée par une multiplicité d'éléments auto-agrippants, non visibles à l'oeil nu, par exemple des boucles

- une pluralité d'éléments complémentaires 5 de fixation réversible, par exemple des pastilles rectangulaires ou circulaires, distinctes de la valise 2, et destinées chacune à coopérer en fixation réversible avec les tapis 41 et 42; plus précisément chaque pastille comporte, d'un côté une face 5a d'adhésion, par exemple collante par simple contact, protégée par un film de protection 6, lui-même non adhésif, et de l'autre côté une face 5b de fixation réversible, auto-agrippante par rapport au tapis 41 et 42 de la valise 2; par exemple, la face de fixation réversible consiste en un coupon textile dont la surface extérieure comporte une multiplicité d'éléments auto-agrippants, non visibles à l'oeil nu, par exemple des crochets.

Ce néssaire est destiné à la fixation réversible de différents objets, au premier rang desquels figurent des emballages unitaires 1, par exemple en carton, présentant chacun une face antérieure la de présentation, par exemple sur laquelle peuvent être lues différentes informations, et une face opposée postérieure 1b, sans valeur informative. Mais ce nécessaire peut aussi servir à la fixation d'objets tels que 7, consistant en un flacon cylindrique transparent laissant apparaître son contenu, ainsi qu'à divers instruments ou matériels, tels que représentés sous les références numériques 8 à 10 à la Figure 4.

L'utilisation du nécessaire de transport selon l'invention, précédemment décrite, s'effectue comme suit.

Après avoir choisi et retenu les objets, dont emballages unitaires et instruments qu'il désire ranger et transporter dans la valise 2, l'utilisateur retient autant de pastilles 5 que d'objets. Après avoir détaché de chaque pastille le film de protection 6, il colle chacune d'entre elles par sa face 5a sur chaque objet retenu. En particulier, s'agissant d'un emballage unitaire, comportant une face de présentation ou d'information la, il colle la pastille 5 sur la face 1b opposée à la face de présentation la, de telle manière qu'il obtient un emballage présentant une face postérieure permettant une fixation réversible directe par simple pression, à l'intérieur de la valise 2, grâce à la face auto-agrippante 5b de l'élément 5 collé.

Tous les objets, notamment emballages unitaires, sont ensuite fixés directement, par leur face rendue auto-agrippante comme précédemment, sur l'un des tapis complémentaires 41 et 42 de fixation réversible.

On obtient ainsi pour l'ensemble des objets auto-agrippés à l'intérieur de la valise 2, une présentation du type de celle représentée à la Figure 4.

La valise représentée à la Figure 4, ne diffère de celle représentée à la Figure 1 que par la substitution aux tapis 41 et 42 de six bandes 45 à 48 de fixation réversible, telles que des rubans, couvrant de manière espacée la face intérieure des parties 21 et 22 de la valise 2.

Comme dit plus haut, la présente invention s'applique plus particulièrement à des trousses d'usage médical, notamment des trousses d'urgence, les emballages unitaires 1 étant des boîtes de médicament, telles que disponibles dans le commerce, et les objets 7, 8 et 9 étant des instruments médicaux ou chirurgicaux, tels que chambre d'installation, lecteur de glycémie, aérosol, etc...

## Revendications

1) Necéssaire de transport de différents produits contenus respectivement dans différents emballages (1) unitaires, par exemple en carton, chaque emballage unitaire comprenant une face de présentation (la) du produit contenu, par exemple une face informative, ledit nécessaire comprenant un contenant (2) dont l'intérieur est accessible au chargement avec les différents produits emballés, grâce à un moyen (3) d'ouverture-fermeture dudit contenant, la surface interne dudit contenant comprenant de manière non détachable des moyens (4) de fixation réversible, tels qu'auto-agrippants, caractérisé en ce que le nécessaire comporte avant utilisation une pluralité d'éléments complémentaires (5) de fixation réversible, tels que pastilles, distincts du contenant (2), comportant chacun d'un côté une face (5a) d'adhésion à la surface d'un emballage (1) unitaire, protégée par un film de protection (6) non adhésif, et de l'autre côté, une face (5b) de fixation réversible, telle qu'auto-agrippante, sur les moyens (2) de fixation réversible du contenant.

2) Nécessaire selon la revendication 1, caractérisé en ce que le contenant (2) comprend un tapis (41,42) de fixation réversible, couvrant au moins l'une des faces intérieures dudit contenant.

**3)** Nécessaire selon la revendication 1, caractérisé en ce que le contenant (2) comprend des bandes (45 à 48) de fixation réversible, telles que rubans, couvrant de manière espacée au moins l'une des faces intérieure dudit contenant (2).

**4)** Nécessaire selon la revendication 1, caractérisé en ce que le contenant (2) est une valise ou mallette de transport.

**5)** Nécessaire selon la revendication 1, prêt à être utilisé, caractérisé en ce que, d'une part les différents éléments de fixation réversible (5) sont fixés sur une face (1b) des différents emballages (1) unitaires, opposée à leur face de présentation (1a), pour obtenir des emballages unitaires (Fig.2) pouvant être fixés directement de manière réversible dans le contenant (2), et d'autre part les différents emballages unitaires (1) sont fixés directement de manière réversible sur les moyens de fixation (4) réversible du contenant (2).

**6)** Nécessaire selon la revendication 5, caractérisé en ce que, d'une part d'autres éléments (5) de fixation réversible sont fixés sur une face d'autres objets (7 à 9), tels qu'instruments, pour obtenir des objets pouvant être fixés directement de manière réversible dans le contenant (2), et d'autre part les différents objets sont fixés directement de manière réversible sur les moyens de fixation (4) réversible du contenant.

**7)** Nécessaire selon l'une quelconque des revendications 5 et 6, caractérisé en ce que le nécessaire est à usage médical, et les emballages (1) sont des boîtes de médicament, telles que disponibles dans le commerce, et les objets fixés (7 à 9) dans le contenant sont des instruments médicaux ou chirurgicaux.

**7)** Nécessaire selon la revendication 7, caractérisé en ce qu'il consiste en une trousse médicale d'urgence.

FIG 1

FIG 2

FIG 3

FIG 4

Office européen
des brevets

**RAPPORT DE RECHERCHE EUROPEENNE**

Numero de la demande

EP    92 42 0220

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y | US-A-4 386 642 (DURBIN)<br>* le document en entier * | 1,5,6 | A45C5/12 |
| A | | 3,4,7,8 | |
| | --- | | |
| Y | EP-A-0 210 536 (YOSHIDA KOGYO)<br>* page 1, ligne 8 - page 2, ligne 9;<br>figure 7 * | 1,5,6 | |
| | --- | | |
| A | US-A-4 940 250 (CORRADO)<br>* colonne 2, ligne 47 - colonne 3, ligne 22; figures 1-3 * | 1,3-6 | |
| | --- | | |
| D,A | FR-A-2 653 312 (BRICCOLA)<br>* page 2, ligne 29 - page 3, ligne 28;<br>figure 1 * | 1,2,4 | |
| | ----- | | |

**DOMAINES TECHNIQUES RECHERCHES (Int. Cl.5 )**

A45C
A44B

Le présent rapport a été établi pour toutes les revendications

| Lieu de la recherche | Date d'achèvement de la recherche | Examinateur |
|---|---|---|
| LA HAYE | 06 OCTOBRE 1992 | SIGWALT C. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

EPO FORM 1503 03.82 (P0402)